(19) Europäisches Patentamt
European Patent Office
Office européen des brevets

(11) Veröffentlichungsnummer: **0 058 927**
**B1**

(12) # EUROPÄISCHE PATENTSCHRIFT

(45) Veröffentlichungstag der Patentschrift: **30.05.84**

(51) Int. Cl.³: **C 07 C 45/75**, C 07 C 47/21, C 07 C 47/22

(21) Anmeldenummer: **82101164.0**

(22) Anmeldetag: **17.02.82**

(54) **Verfahren zur Herstellung von alpha-Alkylacroleinen.**

(30) Priorität: **21.02.81 DE 3106557**

(43) Veröffentlichungstag der Anmeldung: **01.09.82 Patentblatt 82/35**

(45) Bekanntmachung des Hinweises auf die Patenterteilung: **30.05.84 Patentblatt 84/22**

(84) Benannte Vertragsstaaten: **BE CH DE FR GB IT LI**

(56) Entgegenhaltungen:
**FR - A - 2 444 659**
**GB - A - 2 078 748**

(73) Patentinhaber: **BASF Aktiengesellschaft, Carl-Bosch-Strasse 38, D-6700 Ludwigshafen (DE)**

(72) Erfinder: **Merger, Franz, Dr., Max-Slevogt-Strasse 25, D-6710 Frankenthal (DE)**
Erfinder: **Foerster, Hans-Juergen, Dr., Leipziger Strasse 10, D-6712 Bobenheim-Roxheim (DE)**

**EP 0 058 927 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die Erfindung betrifft ein Verfahren zur Herstellung von α-Alkylacroleinen durch Umsetzung von Alkanalen mit Formaldehyd und sekundären Aminen in Gegenwart von Carbonsäuren oder Dicarbonsäuren bzw. Polycarbonsäuren in bestimmten Molverhältnissen in einem pH-Bereich von 2,5 bis 7 und bei einer Temperatur von 0 bis 150°C.

Es ist bekannt, α-Alkylacroleine durch Mannich-Kondensation von n-Alkanalen und Formaldehyd mit Hilfe von sekundären Aminen herzustellen:

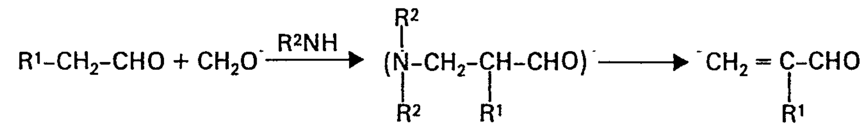


Im allgemeinen werden Temperaturen von über 40°C, Drücke von 1–10 bar und pH-Werte > 3 verwendet. Hierbei kondensieren die Alkanale mit Formaldehyd in $R^2NH$ zur Mannich-Base, deren Spaltung das α-Alkylacrolein ergibt unter Freisetzung des Amins, das vorteilhaft häufig zusammen mit starken Säuren (HCl, HBr, $H_2SO_4$, $H_3PO_4$, Sulfonsäuren) Salze bildet, die die Reaktion katalysieren.

So können Methacrolein bzw. 2-Ethylacrolein aus Propionaldehyd bzw. n-Butyraldehyd und Formaldehyd nach dem in DE-PS 875194 beschriebenen Verfahren hergestellt werden. In den Beispielen werden Monocarbonsäuren wie Buttersäure und Stearinsäure gezeigt. Molverhältnisse von Propionaldehyd/Formaldehyd = 1/1,02 (mol), Propionaldehyd/sek. Amin = 1/0,038, Propionaldehyd/Säure = 1/0,013, sek. Amin/Säure = 3/1, Verweilzeiten von 4–6 Stunden und Siedetemperaturen bis 100°C werden gezeigt. Man arbeitet im allgemeinen bei einem pH-Wert > 7.

Bei einem ähnlichen Verfahren (DE-OS 2855504) zur Herstellung von Methacrolein werden Carbonsäuren, nämlich Ameisensäure, Essigsäure und insbesondere Propionsäure, und als sekundäre Amine Dipropylamin, Methylbutylamin, Ethylbutylamin und insbesondere Di-n-butylamin hervorgehoben. Im Beispiel werden nur 81,7% Ausbeute bei einer Umsetzung bei 30–100°C, 2,5 bar während 120 Minuten erzielt. Molverhältnisse Propionaldehyd/Formaldehyd = 1/1–1,5, Propionaldehyd/sek. Amin = 1/0,02–0,05 (1/0,025) und Propionaldehyd/Säure = 1/0,01–0,02 (1/0,015) werden vorgeschrieben.

Beide Veröffentlichungen zeigen katalytische Mengen Amine in Verbindung mit Carbonsäuren. Durch Umsetzung bei pH > 7 (Überschuss an stark basischem Amin bezüglich RCOOH) erreicht man bei relativ hoher Temperatur noch befriedigende Reaktionsgeschwindigkeit, so dass Dimerisierungen des Alkylacroleins noch keine grosse Rolle spielen. Wie eigene Arbeiten aber zeigen (siehe Vergleichsbeispiele 6 und 7), sind die Ausbeuten kaum reproduzierbar, da man stets in beträchtlichem Umfang neben Methylacrolein auch andere Aldolkondensationsprodukte (2-Methylpentenal und Methylolverbindungen) erhält.

US-PS 2518416 beschreibt die Kondensation von Alkanal und Formaldehyd in geschmolzenem Salz $R^2NH \cdot HX$ (HX = eine Säure mit der Mindestacidität von Trichloressigsäure), bevorzugt in $(CH_3)_2NH \cdot HCl$, bei 120–300°C, vorzugsweise 140 bis 220°C. Beispiel 1 zeigt eine Ausbeute von 51% Ethylacrolein aus n-Butyraldehyd und Formaldehyd bei 200–220°C.

US-PS 2639295 beschreibt die Kondensation von Propionaldehyd/Formaldehyd, im Überschuss-Verhältnis 2-6/1, in Gegenwart von bis zu 0,25 Äquivalenten $R^2NH \cdot HX$ ($R^2NH$ = sek.- Amin; HX = insbesondere HCl, HBr, $H_2SO_4$ und $H_3PO_4$) bei 80 bis 130°C und pH 4-6, wobei praktisch der gesamte Aldehyd als Azeotrop kontinuierlich abdestilliert wird. Das Ausbeute-Optimum (Beispiel 8) von 92,5%, bezogen auf Formaldehyd, erzielt man mit einem Verhältnis von Propionaldehyd/Formaldehyd = 5/1 Mol und einer 10-prozentigen essigsauren Piperidin · HCl-Lösung.

Die Hauptursache für eine Desaktivierung des Katalysators $R^2NH$, einhergehend mit abnehmender Selektivität, liegt in der Methylierung zu $R^2NCH_3$, d.h. zu katalytisch nicht wirksamen tertiären Aminen [Leuckardt-Wallach-Reaktion, siehe Houben-Weyl, Band 11/1 (1957), Seiten 648-654]. US-PS 2639295 empfiehlt daher einen Überschuss an Propionaldehyd, der jedoch wegen des hohen Stoffkreislaufes unökonomisch ist und die höhere Selektivität bezüglich Formaldehyd mit unvermeidlichen Verlusten an Propionaldehyd, u.a. durch Kondensation zu Methylpentenal erkauft. Die vorgeschlagene Regenerierung des Katalysators durch Erhitzen auf 150–300°C ist bezüglich der gebildeten inaktiven tert. Amine $R^2NCH_3$ unwirksam. Der irreversible Verbrauch des Katalysators ist aber nachteilig mitbestimmend für die Ökonomie eines Verfahrens zur Herstellung von α-Alkylacroleinen durch Mannich-Kondensation. Äusserst aufwendig und umweltbelastend ist die Aufarbeitung bzw. Entsorgung verbrauchter Katalysatoren (auf Grund ihres Mineralsäure-Anteils) im Gemisch mit Nebenprodukten der Synthese.

Ein ähnliches Verfahren beschreibt US-PS 2848499 in der kontinuierlichen Kondensation von Propionaldehyd/Formaldehyd/$R^2NH \cdot HX$ im Verhältnis 1/1/2–5 bei 105–120°C unter Druck. Als Säuren werden genannt HCl, $H_2SO_4$, $H_3PO_4$ und Essigsäure. Das einzige Beispiel ist mit HCl beschrieben und zeigt, dass gerade mit einem Verhältnis von Propionaldehyd/Formaldehyd/$(CH_3)_2NH \cdot HCl$ = 1/1,036/2,5 bei 111°C ein Umsatz von 98,1% Propionaldehyd und eine Selektivität von 99,6%, bezogen auf Propionaldehyd bzw.

99%, bezogen auf Formaldehyd, erzielt wird. Das Verfahren arbeitet mit einem hohen Überschuss an Aminsalz, der ebenfalls wegen des hohen Stoffkreislaufes unökonomisch ist; zudem treten auch hier, bei Verwendung des bevorzugten Katalysators Dimethylaminhydrochlorid, die weiter oben beschriebene Probleme bezüglich Aufarbeitung bzw. Entsorgung auf.

Russ. Chem. Rev. 33 (1964) 314 beschreibt die Kondensation von Propionaldehyd/-Formaldehyd$(C_2H_5)_2NH \cdot HCl$ = 1,1/1/1 bei pH 6–7 während 20 Minuten bei 45°C. Die Freisetzung des Methylacroleins bei 95°C ermöglicht eine Produktion ohne wesentliche Nebenproduktbildung. Dagegen erhalte man katalytisch mit 0,1 Mol $R^2NH$/Mol Formaldehyd bei notwendig höherer Temperatur beträchtliche Mengen «Polymere» und geringe Ausbeuten. Eine starke Abhängigkeit vom pH wurde festgestellt, beim Optimum pH 6–7 könne man im Gegensatz zu pH 3–4 (wo man bei 100–120°C umsetzen müsse) bei so tiefer Temperatur arbeiten, dass ohne Polymerisationsverluste hohe Selektivitäten erreicht werden. Es wird ausdrücklich festgestellt, dass «vorgängige Verfahren dagegen weit vom Optimum entfernt sind». Streuende, schwierig reproduzierbare bzw. unbefriedigende Ausbeuten ergeben sich auf jene Weise. Die Autoren unterstreichen daher die Bedeutung der Bedingungen.

In den vier letztgenannten Verfahren setzt man bevorzugt relativ billige, niedermolekulare Amine (mit hohem NH-Anteil) ein. Hiernach erhält man aber durch einfache Destillation α-Alkylacroleine wie Methylacrolein oder 2-Ethylacrolein mit beträchtlichen Amingehalten, so dass für die Weiterverarbeitung in der Regel aufwendige Raffination unerlässlich ist. Zusätzliche Aminverluste beim Aufkonzentrieren des Katalysatorgemisches nach Abtrennung des produzierten Methylacroleins durch Abdestillieren des Reaktionswassers sowie des mit Formaldehyd eingebrachten Wassers, sind umso grösser, je leichter flüchtig das eingesetzte Amin ist.

Alle genannten Verfahren sind bezüglich gleichzeitig optimaler Werte in den Ausbeuten, dem Katalysatoraufwand, dem technisch einfachen Betrieb unbefriedigend; DE-PS 875 194 und DE.OS 28 55 504 verlaufen – bei nicht vernachlässigbarem Aminverbrauch – nicht ausreichend selektiv; US-PS 2 518 416, 2 639 295, 2 848 499 sind verfahrenstechnisch sehr kompliziert (Einsatz hoher Überschüsse einzelner Komponenten, erschwerte Bedingungen) bzw. liefern ebenfalls schlechte Ergebnisse; das russische Verfahren ergibt zwar in technisch einfacher Form gute Ausbeuten, hat aber noch wesentliche Nachteile: Toxizitätsprobleme schliessen die Verwendung des empfohlenen Katalysators $(C_2H_5)_2NH \cdot HCl$ aus; die Produktqualität ist durch den Amingehalt minderwertig; der Katalysatorverbrauch ist erheblich, die Entsorgung anorganisch-organischer Abfallprodukte schwierig, darüber hinaus ist $NR_2H \cdot HCl$ stark korrosiv.

Es wurde nun gefunden, dass α-Alkylacroleine der Formel

$CH_2=C-CHO$ I,
   |
   $R^1$

worin $R^1$ einen aliphatischen Rest bedeutet, durch Umsetzung von Alkanalen der Formel

$R^1-CH_2-CHO$ II,

worin $R^1$ die vorgenannte Bedeutung hat, mit Formaldehyd und sekundärem Amin in Gegenwart von Säure vorteilhaft hergestellt werden, wenn die Umsetzung mit einem Molverhältnis von Ausgangsstoff II zu Formaldehyd von 0,9–1,5/l, bei einem pH-Wert von 2,5–7 und bei einer Temperatur von 0 bis 150°C in Gegenwart von

a) aliphatischen Monocarbonsäuren mit 2–10 Kohlenstoffatomen in einer Menge von 0,05 bis 1,5 Äquivalenten Carbonsäure je Mol Ausgangsstoff II oder

b) Dicarbonsäuren oder Polycarbonsäuren mit 2 bis 10 Kohlenstoffatomen in einer Menge von 0,01 bis 1,5 Äquivalenten Di- bzw. Polycarbonsäure je Mol Ausgangsstoff II als Säuren

und mit sekundären Aminen in einem Verhältnis von 1 bis 2 Äquivalenten Carbonsäure je Äquivalent Amin durchgeführt wird.

Die Umsetzung kann für den Fall der Verwendung von Propionaldehyd durch die folgende Formel wiedergegeben werden:

$CH_3-CH_2-CHO + CH_2O \longrightarrow CH_2=CH-CHO$
                                   |
                                  $CH_3$

Die Erfindung geht von der Beobachtung aus, dass nicht nur die Ausbeute, sondern darüber hinaus noch weitere zum Teil sogar wichtigere Ergebnisse der Umsetzung für ein wertvolles Verfahren in Betracht gezogen werden müssen. Im Hinblick auf den Stand der Technik liefert das Verfahren nach der Erfindung eine Kombination vergleichsweise optimaler Ergebnisse, z.B. hohe Selektivität bezüglich beider Komponenten (Alkanal, Formaldehyd) bei fast quantitativem Umsatz, hohe Raum-Zeit-Ausbeute, hohe Katalysatorleistung Q=Mole α-Alkylacrolein/sek. Amin-Äquivalent, gute Produktqualität (direkte Weiterverarbeitbarkeit), geringer technischer Aufwand, Ausschluss gravierender Toxizitätsprobleme, ökonomisch rationelle, ökologisch unbedenkliche Entsorgung. Alle diese Ergebnisse sind im Hinblick auf die bekannten Verfahren überraschend.

Das Verfahren hebt sich von den bekannten Methoden besonders überraschend durch höhere Katalysatorleistung Q ab. Während sekundäre Amine in Kombination mit Mineralsäuren wie z.B. Schwefelsäure bei Umsetzungen von n-Alkanalen mit Formaldehyd Q ≈ 20 Mole/sek. Amin-Äquivalent ergeben, liefert ihre Kombination mit Di- bzw. Polycarbonsäuren Q ≈ 60 bis 70 mole/sek. Amin-Äquivalent. Es ermöglicht eine technisch und wirtschaftlich vorteilhafte Produktion von α-Alkylacroleinen hoher Reinheit und ist daher u.a. vorzüglich brauchbar zur Produktion von Methacrolein, das bereits als Rohdestillat (mit einem Gehalt von 2–3% Wasser) zur Herstellung von Methacrylsäure durch Oxidation mit $O_2$ sehr gut geeignet ist.

Die Alkanale können mit Formaldehyd in stöchiometrischen Mengen, im Unterschuss oder auch im Überschuss, in einer Menge von 0,9 bis 1,5, vorzugsweise 0,95 bis 1,2, insbesondere 1,0 bis 1,10 Mol Ausgangsstoff II je Mol Formaldehyd umgesetzt werden. Bevorzugte Ausgangsstoffe II und dementsprechend bevorzugte Endstoffe I sind solche, in deren Formeln $R^1$ einen Alkylrest mit 1 bis 8 Kohlenstoffatomen bedeutet. Die vorgenannten Reste können noch durch unter den Reaktionsbedingungen inerte Gruppen, z.B. Alkoxygruppen oder Alkylgruppen mit 1 bis 4 Kohlenstoffatomen, substituiert sein.

So kommen als Ausgangsstoffe II beispielsweise in Frage: Propanal, n-Butanal, 3-Methylbutanal, n-Pentanal, 3-Methylhexanal, 3-Ethylpentanal, 4-Methylhexanal, n-Heptanal, n-Nonanal, n-Decanal.

Das Formaldehyd wird zweckmässig in wässriger, vorteilhaft in 20–60-gewichtsprozentiger Lösung verwendet. Die Dicarbonsäuren und Polycarbonsäuren (darunter vorzugsweise Tricarbonsäuren) können auch aromatische und araliphatische Carbonsäuren sein. Dicarbonsäuren und Polycarbonsäuren sind vorteilhafter als Monocarbonsäuren. Geeignet sind z.B.: Essigsäure, Propionsäure, Methoxyessigsäure, Buttersäuren, Pentan-, Hexan-, Heptan, Octan-, Nonan-, Decansäuren, Pivalinsäure, 2-Ethylbuttersäure, 2-Methylpentansäure, 2-Ethylhexansäure, Isononansäure; Pimelinsäure, Korksäure, Acelainsäure, Sebacinsäure, Nonandicarbonsäure, Decandicarbonsäure, Butantetracarbonsäure, Pentan-1,3,5-tricarbonsäure, 3-Hydroxyglutarsäure, Zuckersäure, α,α'-Dihydroxyadipinsäure, bevorzugt Oxalsäure, Bernsteinsäure, Glutarsäure, Adipinsäure, Äpfelsäure, Weinsäure, Butan-1,2,4-tricarbonsäure, 3-Äthylpentan-1,3,5-tricarbonsäure, Zitronensäure, Trimellitsäure, Butantetracarbonsäure, Pyromellitsäure, Phthalsäure, Terephthalsäure, Isophthalsäure, Fumarsäure und besonders bevorzugt Oxalsäure.

Als Amine kommen zweckmässig solche der Formel

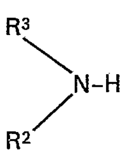 III,

worin $R^3$ und $R^2$ gleich oder verschieden sein können und einen, vorteilhaft mit einem oder mehreren Heteroatomen, bevorzugt mit Hydroxyl und/oder sekundärem bzw. tertiärem Amin substituierten Alkylrest mit 1 bis 12, vorteilhaft 1 bis 10, bevorzugt 1 bis 6 Kohlenstoffatomen bedeuten, $R^2$ und $R^3$ mit dem benachbarten Kohlenstoffatom auch Glieder eines vorteilhaft 5- oder 6-gliedrigen Ringes, der noch ein Stickstoff- oder Sauerstoffatom enthalten kann, in Frage. $R^2$ kann auch den Rest

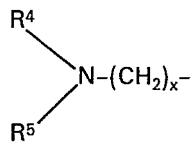 bedeuten,

worin $R^4$ und $R^5$ gleich oder verschieden sein können und jeweils einen, gegebenenfalls durch mehrere, vorteilhaft zwei, bevorzugt eine Hydroxygruppe substituierten Alkylrest mit 2 bis 18, zweckmässig 2 bis 10, bevorzugt 2 bis 6 Kohlenstoffatomen bezeichnen, $R^4$ auch für ein H-Atom stehen kann und x eine Zahl von 2 bis 6 bedeutet, in Frage. Vorteilhaft steht bei Monohydroxyalkylresten die Hydroxygruppe in ω-Stellung. Bevorzugt werden sekundäre Amine mit einem Siedepunkt oberhalb von 130°C. Besonders bevorzugt werden sekundäre Amine mit sehr geringer Flüchtigkeit wie z.B. solche Hydroxyalkylamine, die man z.B. aus Ammoniak oder primären Aminen mit Alkylenoxiden leicht gewinnen kann oder Amine mit zwei oder mehreren Aminogruppen von denen mindestens eine sekundär, die anderen sekundär und/oder tertiär sind.

So kommen als Amine III in Betracht: N-Methyl-, N-Ethyl-, N-Propyl-, N-Isopropyl-, N-Butyl-, N-Isobutyl-, N-sek.-Butyl-, N-tert.-Butyl-, N-Pentyl-, N-Hexyl-, N-Heptyl-, N-Octyl-, N-Nonyl-, N-Decyl-(hydroxyethylamin); entsprechende durch einen vorgenannten Substituenten gleich oder unterschiedlich zweimal substituierte Amine; Piperidin, Morpholin, Pyrrolidin, Piperazin, N-Methylpiperazin; N-Ethyl-, N-Propyl-, N-Isopropyl-, N-Butyl-, N-Isobutyl-, N-sek.-Butyl-, N-tert.-Butyl-, N-Pentyl-, N-Hexyl-, N-Heptyl-, N-Octyl-, N-Nonyl-, N-Decyl-N-Hydroxyethylamin; entsprechende durch einen vorgenannten hydroxylfreien Substituenten und durch die Hydroxypropyl- und Hydroxybutyl-Gruppe substituiertes Amin; gleich oder unterschiedlich durch vorgenannte Hydroxyalkylsubstituenten zweimal substituierte Amine N,N,N'-Triethanolethylendiamin, N,N'-Diethanolethylendiamin und homologe Di- bzw. Tripropanol-Verbindungen bzw. entsprechende Propylen- und Butylendiamin-Verbindungen. Bevorzugt sind Methylhydroxyethylamin, Ethylhydroxyethylamin, Propylhydroxyethylamin, Isopropylhydroxyethylamin, Butylhydroxyethylamin, Isobutylhydroxyethylamin, Methylhydroxypropylamin, Ethylhydroxypropylamin, Propylhydroxypropylamin, Isopropylhydroxypropylamin, Butylhydroxypropylamin, Isobutylhydroxypropylamin, Dihydroxyethylamin, Dihydroxypropylamin, N,N'-Diethanoletylendiamin, Piperazin, N-Methylpiperazin, Dibutylamin.

Man verwendet im Falle von Monocarbonsäuren vorteilhaft zwischen 0,05 bis 1,5, zweckmässig 0,06 bis 1,4, bevorzugt 0,3 bis 1,25, insbesondere 0,6 bis 1,1 Äquivalente Monocarbonsäure und vorteilhaft 0,05 bis 1,5, bevorzugt 0,3 bis 1,25,

insbesondere 0,6 bis 1,1 Äquivalente Amine je Ausgangsstoff II. Man verwendet im Falle von Di- oder Polycarbonsäuren vorteilhaft zwischen 0,01 bis 1,5, bevorzugt 0,05 bis 1,5, insbesondere 0,3 bis 1,25 Äquivalente Di- bzw. Polycarbonsäure und vorteilhaft 0,01 bis 1,5, bevorzugt 0,05 bis 1,5, insbesondere 0,3 bis 1,25 Äquivalente Amine je Ausgangsstoff II. Man setzt mit einem Verhältnis von 1 bis 2, vorzugsweise 1,05 bis 1,8, insbesondere 1,1 bis 1,5 Äquivalenten Carbonsäure je Äquivalent Amin um.

Die Umsetzung wird bei einem pH von 2,5 bis 7, vorzugsweise 3 bis 6,5, insbesondere 3 bis 6, einer Temperatur von 0°C bis 150°C, vorteilhaft 20 bis 130°C, bevorzugt 30 bis 120°C, insbesondere 40 bis 110°C und drucklos oder unter Druck oder Unterdruck, kontinuierlich oder diskontinuierlich durchgeführt. Der Wassergehalt des Reaktionsgemisches beträgt zweckmässig 20 bis 80 Gewichtsprozent, bevorzugt 20 bis 40 Gewichtsprozent im Ausgangsgemisch.

Die Reaktion kann wie folgt durchgeführt werden: Ein Gemisch von Ausgangsstoff II, Amin III, Formaldehyd, Wasser, Säure wird während 1–300 Minuten, in der Regel 5–120 Minuten, vorzugsweise 10–90 Minuten bei der Reaktionstemperatur gehalten. Dann wird aus dem Reaktionsgemisch der Endstoff in üblicher Weise, z.B. durch Phasentrennung und/oder Destillation abgetrennt. Man kann das Verfahren in verschiedenen Reaktoren (Rührkessel, Rohrreaktoren) diskontinuierlich oder kontinuierlich durchführen. So kann man diskontinuierlich zur Herstellung von α-Alkylacrolein in das aus sek. Amin und einer Carbonsäure vorbereitete, 30–60 Gewichtsprozent Wasser enthaltende Katalysatorgemisch zunächst Formaldehyd einbringen und danach Ausgangsstoff II zuführen oder Formaldehyd und Alkanal gleichzeitig, gemeinsam oder parallel, zulaufen und unter den definierten Bedingungen abreagieren lassen. Aus dem Gemisch ist der Endstoff I durch Phasentrennung und/oder durch diskontinuierliche oder kontinuierliche Destillation isolierbar.

Kontinuierlich führt man die Kondensation zweckmässig in Kaskaden von 2–3 Rührkesseln durch, indem man dem umlaufenden Katalysatorgemisch Formaldehyd und Alkanal gleichzeitig zuführt und aus dem Produktgemisch das α-Alkylacrolein und das mit Formaldehyd eingebrachte sowie durch die Reaktion gebildete $H_2O$ zusammen oder nacheinander kontinuierlich abdestilliert. Aus dem Katalysatorgemisch zieht man einen dem Verbrauch an Amin III entsprechenden Anteil ab und führt diesen der Aufbereitung bzw. Entsorgung, die durch rückstandloses Verbrennen erfolgen kann, zu.

Die nach dem Verfahren herstellbaren α-Alkylacroleine sind wertvolle Ausgangsstoffe für Farbstoffe, Pharmazeutika und Schädlingsbekämpfungsmittel. Durch Oxidation von Methacrolein gelangt man zu den entsprechenden Methacrylsäuren und Methacrylsäureestern, die bei der Herstellung von Kunststoffen, Acrylgläsern, Formmassen, Profilen, Lacken, Schmierölen, Klebern, Textilhilfsmitteln wertvoll sind. Bezüglich der Verwendung wird auf vorgenannte Literatur und Ullmanns Encyklopädie der technischen Chemie (4. Ausgabe), Band 16, Seiten 609 bis 614, verwiesen. Die in den folgenden Beispielen aufgeführten Teile bedeuten Gewichtsteile.

Beispiel 1

Mit 1125 Teilen einer 40-gewichtsprozentigen Oxalsäure (5 Mol) und 1050 Teilen (10 Mol) Diethanolamin wird das Aminsalz hergestellt. Anschliessend fügt man bei 20°C 750 Teile einer 40-gewichtsprozentigen Formaldehyd-Lösung sowie 580 Teile Propionaldehyd hinzu (10 Mol) und hält das Reaktionsgemisch eine Stunde bei einer Temperatur von 40 bis 50°C. Der rohe Endstoff enthält 2,6 Gewichtsprozent Wasser und 0,8 Gewichtsprozent organische Verunreinigungen. Durch azeotropische Destillation erhält man 660 Teile (94,3% der Theorie) Methacrolein vom Kp 68°C (1013 mbar).

Beispiel 2

Mit 2281 Teilen einer 16-gewichtsprozentigen wässrigen Adipinsäure (2,5 Mol) und 215 Teilen Piperazin (2,5 Mol) wird das Aminsalz hergestellt. Anschliessend fügt man bei 20°C 375 Teile einer 40-gewichtsprozentigen Formaldehydlösung sowie 290 Teile Propionaldehyd hinzu (5 Mol) und hält das Reaktionsgemisch eine Stunde bei einer Temperatur von 40–50°C. Der rohe Endstoff enthält 2,5 Gewichtsprozent Wasser und 0,8 Gewichtsprozent organische Verunreinigungen. Durch Destillation erhält man 320 Teile (91,4% der Theorie) Methacrolein vom Kp 68°C (1013 mbar).

Beispiel 3

Mit 1181 Teilen einer 40-gewichtsprozentigen Oxalsäure (5,25 Mol) und 1050 Teilen (10 Mol) Diethanolamin wird das Aminsalz hergestellt. Anschliessend fügt man bei 20°C 750 Teile einer 40-gewichtsprozentigen Formaldehydlösung sowie 720 Teile n-Butylraldehyd hinzu (10 Mol) und hält das Reaktionsgemisch eine Stunde bei einer Temperatur von 40 bis 50°C. Der rohe Endstoff enthält 2,2 Gewichtsprozent Wasser und 1,2 Gewichtsprozent organische Verunreinigungen. Durch Destillation erhält man 801 Teile (95,4% der Theorie) Äthylacrolein.

Beispiel 4 (Vergleichsbeispiel)

Mit 245 Teilen 40-gewichtsprozentiger Schwefelsäure und 210 Teilen Diethanolamin (2 Mol) wird das Aminsalz hergestellt. Anschliessend fügt man bei 20°C 300 Teile 40-gewichtsprozentigen Formaldehyd sowie 232 Teile Propionaldehyd (4 Mol) hinzu und hält das Reaktionsgemisch 2 Stunden bei 40–50°C. Anschliessend wird zunächst das Methacrolein und im Anschluss daran das mit dem Formaldehyd eingebrachte sowie das während der Reaktion entstandene Wasser abdestilliert. Der so wiederhergestellten Katalysatorlösung werden wiederum 300 Teile 40-gewichtsprozentiger Formaldehyd sowie 232 Teile Propionaldehyd zugefügt und 2 Stunden bei 40

bis 50°C belassen. Nach Destillation von Methacrolein und Wasser wird dieselbe Katalysatorlösung erneut eingesetzt. Dieser Vorgang wird so lange wiederholt, bis die bei der Destillation übergehende organische Phase einen Gehalt von <90% an Methacrolein aufweist. Insgesamt werden so (umgerechnet auf 100-prozentiges Methacrolein) 3000 Teile Methacrolein erhalten, das entspricht einer Methacrolein-Menge von 21,4 Molen/sek. Amin-Äquivalent.

Beispiel 5

Mit 225 Teilen 40-gewichtsprozentiger Oxalsäure und 210 Teilen Diethanolamin (2 Mol) wird das Aminsalz hergestellt. Anschliessend fügt man bei 20°C 300 Teile 40-gewichtsprozentigen Formaldehyd sowie 232 Teile Propionaldehyd hinzu (4 Mol) und hält das Reaktionsgemisch 2 Stunden bei 40–50°C. Anschliessend wird zunächst das Methacrolein und im Anschluss daran das mit dem Formaldehyd eingebrachte sowie das während der Reaktion entstandene Wasser abdestilliert. Der so wiederhergestellten Katalysatorlösung werden wiederum 300 Teile 40-gewichtsprozentiger Formaldehyd sowie 232 Teile Propionaldehyd zugefügt und 2 Stunden bei 40–50°C belassen. Nach Destillation von Methacrolein und Wasser wird dieselbe Katalysatorlösung erneut eingesetzt. Dieser Vorgang wird so lange wiederholt, bis die bei der Destillation übergehende organische Phase einen Gehalt von <90% an Methacrolein aufweist. Insgesamt werden so (umgerechnet auf 100-prozentiges Methacrolein) 8880 Teile Methacrolein erhalten, das entspricht einer Methacrolein-Menge von 63,4 Molen/sek. Amin-Äquivalent.

Beispiel 6 (Vergleichsbeispiel)

In einem Autoklaven werden 622 Teile Propionaldehyd (10,7 Mol), 12 Teile Propionsäure (0,16 Mol), 1180 Teile 30-gewichtsprozentiger Formaldehyd (11,8 Mol) sowie 35 Teile Di-n-butylamin (0,25 Mol) unter Stickstoffatmosphäre gemischt, wobei die Temperatur auf 28°C ansteigt. Dann wird bei 100°C eine Stunde gerührt, wobei sich ein Druck von 2,4 bar einstellt. Nach Abkühlung wird das Reaktionsgemisch in zwei Phasen aufgetrennt und jede Phase für sich destillativ aufgearbeitet. Der rohe Endstoff enthält 2,5 Gewichtsprozent Wasser und 7,3 Gewichtsprozent organische Verunreinigungen. Durch Destillation erhält man 588 Teile (78,5% der Theorie) Methacrolein vom Kp 68°C (1013 mbar).

Beispiel 7 (Vergleichsbeispiel)

Zu 350 Teilen 30-gewichtsprozentigem Formaldehyd und 195 Teilen Propionaldehyd werden 40 Teile Natriumchlorid und 4 Teile n-Buttersäure gegeben und innerhalb von 2 Stunden 6,5 Teile Piperidin hinzugegeben. Anschliessend wird weitere 3 Stunden am Rückfluss gekocht. Es lassen sich nach dieser Zeit 112 Teile organisches Material bis zu einem Siedepunkt von 100°C abdestillieren. Der rohe Endstoff enthält 2,6 Gewichtsprozent Wasser und 19 Gewichtsprozent organische Verunreinigungen. Durch Destillation erhält man 88 Teile (37,4% der Theorie) Methacrolein vom Kp 68°C (1013 mbar).

Beispiel 8 (Vergleichsbeispiel)

Mit 245 Teilen 40-gewichtsprozentiger Schwefelsäure und 210 Teilen Diethanolamin (2 Mol) wird das Aminsalz hergestellt. Anschliessend fügt man bei 20°C 300 Teile 40-gewichtsprozentigen Formaldehyd sowie 288 Teile n-Butyraldehyd (4 Mol) hinzu und hält das Reaktionsgemisch 2 Stunden bei 40–50°C. Anschliessend wird zunächst das 2-Ethylacrolein und im Anschluss daran das mit dem Formaldehyd eingebrachte sowie das während der Reaktion entstandene Wasser abdestilliert. Der so wiederhergestellten Katalysatorlösung werden wiederum 300 Teile 40-gewichtsprozentiger Formaldehyd sowie 288 Teile n-Butyraldehyd zugefügt und 2 Stunden bei 40 bis 50°C belassen. Nach Destillation von 2-Ethylacrolein und Wasser wird dieselbe Katalysatorlösung erneut eingesetzt. Dieser Vorgang wird so lange wiederholt, bis die bei der Destillation übergehende organische Phase einen Gehalt von <90% an 2-Ethylacrolein aufweist. Insgesamt werden so (umgerechnet auf 100-prozentiges 2-Ethylacrolein) 3225 Teile 2-Ethylacrolein erhalten, das entspricht einer 2-Ethylacrolein-Menge von 19,2 Molen/sek. Amin-Äquivalent.

Beispiel 9

Mit 1500 Teilen einer 40-gewichtsprozentigen Essigsäure (10 Mol) und 1050 Teilen Diethanolamin (10 Mol) wird das Aminsalz hergestellt. Anschliessend fügt man bei 20°C 750 Teile einer 40-gewichtsprozentigen Formaldehydlösung (10 Mol) sowie 580 Teile Propionaldehyd (10 Mol) hinzu und hält das Reaktionsgemisch eine Stunde bei einer Temperatur von 40–50°C. Der rohe Endstoff enthält 2,6 Gewichtsprozent Wasser und 1,8 Gewichtsprozent organische Verunreinigungen. Durch Destillation erhält man 643 Teile (91,8% der Theorie) Methacrolein vom Kp 68°C.

Beispiel 10

Mit 3600 Teilen einer 40-gewichtsprozentigen 2-Ethylhexansäure (10 Mol) und 430 Teilen Piperazin (5 Mol) wird das Aminsalz hergestellt. Anschliessend fügt man bei 20°C 750 Teile einer 40-gewichtsprozentigen Formaldehydlösung (10 Mol) sowie 580 Teile Propionaldehyd (10 Mol) hinzu und hält das Reaktionsgemisch eine Stunde bei einer Temperatur von 40 bis 50°C. Der rohe Endstoff enthält 2,8% Wasser und 1,65% organische Verunreinigungen. Durch Destillation erhält man 672 Teile (96% der Theorie) Methacrolein vom Kp 68°C (1013 mbar).

Beispiel 11

Mit 2420 Teilen einer 40-gewichtsprozentigen Isobuttersäure (1 Mol) und 430 Teilen Piperazin (5 Mol) wird das Aminsalz hergestellt. Anschliessend fügt man bei 20°C 750 Teile einer 40-gewichtsprozentigen Formaldehydlösung (10 Mol) sowie 580 Teile Propionaldehyd (10 Mol) hinzu

und hält das Reaktionsgemisch eine Stunde bei einer Temperatur von 40–50°C. Der rohe Endstoff enthält 2,6% Wasser und 1,45% organische Verunreinigungen. Durch Destillation erhält man 666 Teile (93,7% der Theorie) Methacrolein vom Kp 68°C (1013 mbar).

Beispiel 12

Mit 1850 Teilen einer 40-gewichtsprozentigen Propionsäure (10 Mol) und 1290 Teilen Dibutylamin (10 Mol) wird das Aminsalz hergestellt. Anschliessend fügt man bei 20°C 750 Teile einer 40-gewichtsprozentigen Formaldehydlösung (10 Mol) sowie 720 Teile n-Butyraldehyd (10 Mol) hinzu und hält das Reaktionsgemisch eine Stunde bei einer Temperatur von 40–50°C. Der rohe Endstoff enthält 2,2 Gewichtsprozent Wasser und 1,2% organische Verunreinigungen. Durch Destillation erhält man 795 Teile (94,6% der Theorie) α-Ethylacrolein vom Kp 92°C.

Beispiel 13

Mit 2900 Teilen 40-gewichtsprozentiger 2-Methylpentansäure (10 Mol) und 750 Teilen Methylhydroxyethylamin (10 Mol) wird das Aminsalz hergestellt. Anschliessend fügt man bei 20°C 750 Teile 40-gewichtsprozentige Formaldehydlösung (10 Mol) sowie 860 Teile 3-Methylbutanal (10 Mol) hinzu und hält das Reaktionsgemisch 2 Stunden bei einer Temperatur von 40 bis 50°C. Der rohe Endstoff enthält 2,4 Gewichtsprozent Wasser und 2,8 Gewichtsprozent organische Verunreinigungen. Durch Destillation erhält man 902 g Isopropylacrolein (92% der Theorie) vom Kp 108°C.

Beispiel 14

Mit 1500 Teilen einer 40-gewichtsprozentigen Essigsäure (10 Mol) und 430 Teilen Piperazin (5 Mol) wird das Aminsalz hergestellt. Anschliessend fügt man bei 20°C 750 Teile einer 40-gewichtsprozentigen Formaldehydlösung (10 Mol) sowie 1000 Teile n-Hexanal (10 Mol) hinzu und hält das Reaktionsgemisch 3 Stunden bei 40–50°C. Der rohe Endstoff enthält 0,6 Gewichtsprozent Wasser und 2,2 Gewichtsprozent organische Verunreinigungen. Durch Destillation erhält man 1047 Teile (93,5% der Theorie) n-Butylacrolein vom Kp 125°C (1013 mbar).

Beispiel 15

Mit 973,3 Teilen 15-gewichtsprozentigen Adipinsäure und 86 Teilen Piperazin (1 Mol = 2 Äquivalente) wird das Aminsalz hergestellt. Anschliessend fügt man bei 20°C 300 Teile 40-gewichtsprozentigen Formaldehyd sowie 288 Teile n-Butyraldehyd hinzu (4 Mol) und hält das Reaktionsgemisch 2 Stunden bei 40–50°C. Anschliessend wird zunächst das 2-Ethylacrolein und im Anschluss daran das mit dem Formaldehyd eingebrachte sowie das während der Reaktion entstandene Wasser abdestilliert. Der so wiederhergestellten Katalysatorlösung werden wiederum 300 Teile 40-gewichtsprozentiger Formaldehyd sowie 288 Teile n-Butyraldehyd zugefügt und 2 Stunden bei 40–50°C belassen. Nach Destillation von 2-Ethylacrolein und Wasser wird dieselbe Katalysatorlösung erneut eingesetzt. Dieser Vorgang wird so lange wiederholt, bis die bei der Destillation übergehende organische Phase einen Gehalt von <90% an 2-Ethylacrolein aufweist. Insgesamt werden so (umgerechnet auf 100-prozentiges 2-Ethylacrolein) 11 190 Teile 2-Ethylacrolein erhalten, das entspricht einer 2-Ethylacrolein-Menge von 66,2 Molen/sek. Amin-Äquivalent.

Beispiel 16

Mit 1600 Teilen einer 40-gewichtsprozentigen Glutarsäure (5 Mol) und 1290 Teilen Dibutylamin (10 Mol) wird das Aminsalz hergestellt. Anschliessend fügt man bei 20°C 750 Teile einer 40-gewichtsprozentigen Formaldehydlösung sowie 1000 Teile n-Hexanal (10 Mol) hinzu und hält das Reaktionsgemisch 3 Stunden bei einer Temperatur von 40–50°C. Der rohe Endstoff enthält 0,6 Gewichtsprozent Wasser und 3,1 Gewichtsprozent organische Verunreinigungen. Durch Destillation erhält man 1038 Teile (92,7% der Theorie) n-Butylacrolein vom Kp 125°C (1013 mbar).

Beispiel 17

In eine aus 2 Rührgefässen bestehende Kaskade werden pro Stunde 986,7 Teile einer 60-gewichtsprozentigen wässrigen Oxalsäure/Diethanolamin-Salz-Lösung (2 Mole Oxalsäure und 4 Mole Diethanolamin pro Stunde), 300 Teile einer 40-gewichtsprozentigen Formaldehydlösung sowie 232 Teile Propionaldehyd (4 Mol) gepumpt. Die Gefässe sind auf 50°C temperiert, die Verweilzeit beträgt 1,27 Stunden. Dem zweiten Gefäss werden pro Stunde 1518,7 Teile entnommen und kontinuierlich destilliert, wobei pro Stunde 273,5 Teile organisches Material und 254 Teile Wasser entnommen werden. Die auf ihren ursprünglichen Stickstoffgehalt aufkonzentrierte Katalysatorlösung wird zurückgeführt. Der rohe Endstoff enthält 2,4 Gewichtsprozent Wasser und 1,2 Gewichtsprozent organische Verunreinigungen. Durch Destillation erhält man pro Stunde 263,7 Teile (94,2% der Theorie) Methacrolein vom Kp 68°C (1013 mbar).

**Patentanspruch**

Verfahren zur Herstellung von α-Alkylacroleinen der Formel

$$CH_2=\underset{\displaystyle R^1}{\underset{|}{C}}-CHO \qquad I,$$

worin $R^1$ einen aliphatischen Rest bedeutet, durch Umsetzung von Alkanalen der Formel

$$R^1-CH_2-CHO \qquad II,$$

worin $R^1$ die vorgenannte Bedeutung besitzt, mit Formaldehyd und sekundärem Amin in Gegenwart von Säure, dadurch gekennzeichnet, dass die Umsetzung mit einem Molverhältnis von Ausgangsstoff II zu Formaldehyd von 0,9–1,5/l, bei einem pH-Wert von 2,5–7 und bei einer Temperatur von 0–150°C in Gegenwart von

a) aliphatischen Monocarbonsäuren mit 2–10 Kohlenstoffatomen in einer Menge von 0,05 bis 1,5 Äquivalenten Carbonsäure je Mol Ausgangsstoff II oder
b) Dicarbonsäuren oder Polycarbonsäuren mit 2 bis 10 Kohlenstoffatomen in einer Menge von 0,01 bis 1,5 Äquivalenten Di- bzw. Polycarbonsäure je Mol Ausgangsstoff II als Säuren

und mit sekundären Aminen in einem Verhältnis von 1–2 Äquivalenten Carbonsäure je Äquivalent Amin durchgeführt wird.

**Revendication**

Procédé de préparation d'α-alkyl-acroléines de la formule

$$\underset{\displaystyle R^1}{CH_2=\overset{}{C}}-CHO \qquad I,$$

dans laquelle $R^1$ représente un groupe aliphatique, par réaction d'alcanals de la formule

$$R^1-CH_2-CHO \qquad II,$$

dans laquelle $R^1$ possède la signification définie, avec le formaldéhyde et une amine secondaire en présence d'un acide, caractérisé en ce que la réaction est effectuée à un pH de 2,5 à 7, à une température de 0 à 150°C et avec un rapport molaire entre le composé de départ II et le formaldéhyde de 0,9:1 à 1,5:1 en présence d'acides choisis parmi:

a) les acides mono-carboxyliques aliphatiques en $C_2$ à $C_{10}$ en une proportion de 0,05 à 1,5 équivalent d'acide carboxylique par mole de composé de départ II ou
b) les acides di-carboxyliques ou poly-carboxyliques en $C_2$ à $C_{10}$ en une proportion de 0,01 à 1,5 équivalent d'acide di- ou poly-carboxylique par mole de composé de départ II,

et d'amines secondaires avec un rapport de 1 à 2 équivalents d'acide carboxylique par équivalent d'amine.

**Claim**

A process for the preparation of an α-alkylacrolein of the formula

$$\underset{\displaystyle R^1}{CH_2=\overset{}{C}}-CHO \qquad I,$$

where $R^1$ is an aliphatic radical, by reacting an alkanal of the formula

$$R^1-CH_2-CHO \qquad II,$$

where $R^1$ has the above meaning, with formaldehyde and a secondary amine in the presence of an acid, wherein the reaction is carried out with a molar ratio of starting material II to formaldehyde of 0.9–1.5:1, at a pH of 2.5–7 and at from 0 to 150°C in the presence of

a) from 0.05 to 1.5 equivalents of an aliphatic monocarboxylic acid of 2 to 10 carbon atoms per mole of starting material II or
b) from 0.01 to 1.5 equivalents of a dicarboxylic or polycarboxylic acid of 2 to 10 carbon atoms per mole of starting material II, as the acid, and in the presence of a secondary amine, using an equivalent ratio of carboxylic acid to amine of 1–2:1.